# EUROPÄISCHE PATENTSCHRIFT

(11) **EP 1 483 022 B1**
(45) Veröffentlichungstag und Bekanntmachung des Hinweises auf die Patenterteilung: **13.02.2008**
(21) Anmeldenummer: 02729993.2
(22) Anmeldetag: 12.03.2002
(51) Int. Cl.: A61N 5/10

(54) **VORRICHTUNG ZUR DURCHFÜHRUNG UND VERIFIKATION EINER THERAPEUTISCHEN BEHANDLUNG SOWIE ZUGEHÖRIGES COMPUTERPROGRAMM**
DEVICE FOR PERFORMING AND VERIFYING A THERAPEUTIC TREATMENT AND CORRESPONDING COMPUTER PROGRAM
DISPOSITIF POUR METTRE EN OEUVRE ET VERIFIER UN TRAITEMENT THERAPEUTIQUE ET PROGRAMME INFORMATIQUE ASSOCIES

(43) Veröffentlichungstag der Anmeldung: 08.12.2004
(73) Patentinhaber: Deutsches Krebsforschungszentrum Stiftung des öffentlichen Rechts, 69120 Heidelberg (DE)
(72) Erfinder: HESSE, Bern-Michael, 64756 Mossautal (DE)
(74) Vertreter: Weber, Walter
(86) Internationale Anmeldenummer: PCT/EP2002/002693
(87) Internationale Veröffentlichungsnummer: WO 2003/076016

(56) Entgegenhaltungen:
- WO-A-00/07669
- WO-A-02/22210
- US-A- 5 233 990
- US-B1- 6 307 914

## Beschreibung

Die Erfindung betrifft eine Vorrichtung zur Durchführung und Verifikation einer therapeutischen Bestrahlung, die eine Strahlenquelle für einen energiereichen Strahl und eine Einrichtung zur Modulation des energiereichen Strahls auf der Gantry eines Bestrahlungsgeräts aufweist, wobei für die Verifikation eine Strahlenquelle für einen Röntgenstrahl im Verhältnis zu einem Zielvolumen der Strahlenquelle für den energiereichen Strahl derart gegenüberliegend angeordnet ist, daß die Richtungen der Strahlen im wesentlichen entgegengesetzt sind, ein Medium zur Erfassung des Röntgenstrahls bezüglich der Richtung desselben hinter dem Zielvolumen und ein Medium zur Erfassung des energiereichen Strahls, bezüglich der Richtung dieses Strahls vor dem Zielvolumen angeordnet sind. Die Erfindung betrifft außerdem ein Computerprogramm zum Betrieb dieser Vorrichtung.

Eine derartige Vorrichtung ist aus der US 5, 233, 990 bekannt. Bei dieser Vorrichtung ist vorgesehen, daß sich der Therapiestrahl und der Röntgenstrahl auf einem als Schirm ausgebildeten Medium abbilden, wodurch vergleichbar wird, ob die Begrenzung des Therapiestrahls durch Abschirmungsblöcke mit dem ebenfalls abgebildeten Röntgenbild des Zielvolumens übereinstimmt. Ein entsprechendes Verfahren zur Steuerung dieser Vorrichtung ist aus dieser Schrift ebenfalls bekannt und die Möglichkeit des Einsatzes eines Computerprogramms erwähnt. Mit dieser Vorrichtung können jedoch nur die Außenkonturen des Therapiestrahls mit den Außenkonturen dies Zielvolumens verglichen werden. Es ist keine räumliche Erfassung, keine Intensitätserfassung der Applikation unterschiedlich stark zu bestrahlender Bereiche und keine Erfassung der Anatomie des Bestrahlungsumfeldes möglich. Gerade Intensität und Umfeld sind jedoch wichtige Informationen, wenn Strukturen mit niedrigem Kontrast wie ein Tumor und Risikoorgane nahe beieinander liegen und deshalb eine exakte Überwachung der Übereinstimmung der Applikation mit der Lage und Anatomie des Zielvolumens sowie eine Überwachung einer kritischen Umgebung des Zielvolumens erforderlich ist.

Der Erfindung liegt daher die Aufgabe zugrunde, eine Strahlenbehandlung verfügbar zu machen, die neben der Kontur des Therapiestrahls auch eine exakte Verifikation von Bereichen unterschiedlicher Bestrahlungsintensitäten und der gesamten dreidimensionalen Anatomie des Zielvolumens und seines Umfeldes, insbesondere bei angrenzenden Risikoorganen, ermöglicht.

Bezüglich einer Vorrichtung der eingangs genannten Art wird die Aufgabe erfindungsgemäß dadurch gelöst, daß das Medium zur Erfassung von Bereichen unterschiedlicher Bestrahlungsdosis des energiereichen Strahls ausgebildet ist und daß eine Steuerung mit den Medien zur Erfassung der Strahlen, mit der Einrichtung zur Modulation des energiereichen Strahls, mit einem Antrieb für die Einstellung der Lage des Patiententisches und mit den Strahlenquellen verbunden, mit einem Behandlungsplan ladbar und derart eingerichtet ist, daß sie die Gantry und die vorgenannten Elemente in der Weise steuert, daß
a) vor der Applikation des energiereichen Strahls eine räumliche Erfassung der Anatomie und Lage des Patienten im Bereich des Zielvolumens durch den Röntgenstrahl erfolgt, indem dieser aus verschiedenen Richtungen auf diesen Bereich gerichtet wird,
b) die erfaßte Anatomie und Lage des Patienten mit dem Behandlungsplan verglichen und die Patientenposition und/oder der Behandlungsplan gegebenenfalls korrigiert wird,
c) der energiereiche Strahl aus einer ersten Richtung appliziert und dabei bezüglich seiner Form und Bereichen unterschiedlicher Bestrahlungsdosis erfaßt wird,
d) in einer Sendepause des energiereichen Strahls eine Erfassung von mindestens einem Teilbereich des Zielvolumens mit der allernächsten Umgebung durch den Röntgenstrahl erfolgt,
e) die Röntgenaufnahme mit dem erfaßten applizierten energiereichen Strahl verglichen und der Behandlungsplan gegebenenfalls korrigiert wird,
f) die Schritte c), d) und e) bis zur Erreichung der durch den Behandlungsplan für die erste Bestrahlungsrichtung vorgeschriebenen Applikation wiederholt werden,
g) die Schritte c) bis f) für alle im Behandlungsplan vorgesehenen Bestrahlungsrichtungen wiederholt werden.

Das erfindungsgemäße Computerprogramm ist derart ausgebildet, daß es die Steuerung der Vorrichtung zur Durchführung der oben genannten Funktionen befähigt. Es kann auf einem fest eingebauten Speicher der Steuerung abgelegt oder mittels eines Datenträgers oder online für die Steuerung verfügbar gemacht werden. Das Steuerungsverfahren dient in derselben Weise dem Betriebsablauf der erfindungsgemäßen Vorrichtung.

Durch die erfindungsgemäße Vorrichtung, sowie das erfindungsgemäße Computerprogramm wird es möglich, der Durchführung und Verifikation einer therapeutischen Bestrahlung einen Behandlungsplan zugrundezulegen, der die Applikationen im dreidimensionalen Raum festgelegt hat.

Im ersten Schritt wird durch den Röntgenstrahl erfaßt, ob der Patient entsprechend dieser dreidimensionalen Planung positioniert ist, wobei sowohl die Position als auch die momentane Anatomie des Patienten durch die Erfassung mit dem Röntgenstrahl aus verschiedenen Richtungen im dreidimensionalen Bereich erfaßt, überprüft und korrigiert werden kann. Bei starken Abweichungen ist es selbstverständlich auch möglich, für eine solche Korrektur die Behandlung zu einem späteren Zeitpunkt wieder aufzunehmen.

In den folgenden Schritten der Applikation werden die Sendepausen des energiereichen Strahls - solche sind, da dieser gepulst ist, in der Regel immer vorhanden - genutzt, um die oben genannten Gegebenheiten zu überprüfen und eine laufende Korrektur und Überprüfung durchzuführen, die sich innerhalb so kurzer Zeiträume vollziehen kann, daß selbst kurzfristige anatomische Veränderungen, wie sie beispielsweise durch Herzschlag und Atmung oder Muskelanspannungen verursacht werden, berücksichtigt werden können. Diese Überprüfung findet für jede einzelne Applikationsrichtung des Therapiestrahls vielfach statt, so daß Fehlbestrahlungen weitgehend ausgeschlossen werden können.

Für diese Überprüfung sieht die Erfindung vor, daß in den Sendepausen des Therapiestrahls mindestens ein Teilbereich des Zielvolumens mit der allernächsten Umgebung durch den Röntgenstrahl erfaßt wird. Es kann, um die Korrektur so zeitnah wie möglich, also möglichst schon bei der auf die Pause folgenden Applikation vornehmen zu können, nur ein kritischer Bereich erfaßt, verifiziert und korrigiert werden. Ein solcher kritischer Bereich könnte beispielsweise ein Tumorrand sein, der an einen Risikobereich, wie das Rückenmark, grenzt. Dann muß der Bereich Tumorrand und Rand des Rückenmarks besonders sorgfältig und exakt überprüft und korrigiert werden.

Insbesondere wird auch - ebenfalls im dreidimensionalen Raum - die unterschiedliche Bestrahlungsdosis für verschiedene Bereiche des Zielvolumens erfaßt und gleichfalls anhand des Behandlungsplans mit der aktuellen Lage und Anatomie verglichen und gegebenenfalls korrigiert. In dieser Überprüfung kann ebenfalls der Bereich um das Zielvolumen einbezogen mit dem Behandlungsplan verglichen und für die Verifikation und Korrektur ständig berücksichtigt werden. Insbesondere muß das gesamte Behandlungsvolumen betrachtet werden, dies ist das gesamte von Strahlen durchsetzte Gewebe. Auch dort müssen Risikoorgane so in Betracht gezogen werden, daß sie unterhalb einer festgelegten Bestrahlungsdosis bleiben. Diese Bestrahlungsdosis und Umfeldüberwachung ist insbesondere dann wichtig, wenn die Bestrahlung in der Nähe lebenswichtiger Organe vorgenommen werden muß, deren Strahlenbelastung auf ein Minimum zu begrenzen ist.

Wesentlich für die Erfindung ist, daß diese Überprüfungen und Korrekturen auf der Grundlage der im ersten Schritt erfaßten dreidimensionalen Gegebenheiten erfolgen, wodurch eine wesentlich größere Exaktheit wie bei dem rein zweidimensionalen Vergleich erzielt wird, den der eingangs genannte Stand der Technik vorschlägt.

Die folgenden Weiterbildungen betreffen die erfindungsgemäße Vorrichtung, sowie das Computerprogramm zum Betrieb der Vorrichtung. Ebenfalls offenbart ist ein entsprechendes Steuerungsverfahren.

Diese sind vorzugsweise derart ausgebildet, daß der laufenden Verifikation eine zeitnahe dreidimensionale Erfassung des Bereichs des Zielvolumens zugrunde liegt. Zeitnah bedeutet, daß nicht nur vor der Applikation, sondern auch während derselben eine dreidimensionale Erfassung des Bereichs des Zielvolumens stattfindet. Dies ist dadurch möglich, daß der Röntgenstrahl in den Sendepausen des energiereichen Strahls aus verschiedenen Richtungen, jedoch innerhalb eines so kleinen Bereichs, daß er der Richtung der Strahlen des energiereichen Strahls noch im wesentlichen entgegengesetzt ist, auf mindestens einen Teilbereich des Zielvolumens mit allernächster Umgebung desselben gerichtet wird, um durch diese aus verschiedenen Richtungen erfaßten Daten auch den vorgenannten Erfassungsbereich dreidimensional zu erfassen und zeitnah für die Verifikation zu berücksichtigen.

Diese verschiedenen Richtungen können auf unterschiedliche Weise festgelegt und technisch realisiert werden. Ein Vorschlag sieht vor, daß die Strahlungsquelle für den Röntgenstrahl derart ausgebildet ist, daß sie eine Kreisbewegung in einer Ebene, die um eine durch das Zielvolumen zur Strahlungsquelle des energiereichen Strahls führenden Achse angeordnet ist, vollzieht. Um die entsprechende Verarbeitung dieser Daten vornehmen zu können, ist ein entsprechende Steuerungsverfahren erforderlich und ein Computerprogramm, das zur Auswertung dieser Daten der Röntgenaufnahmen ausgebildet ist. Die Kreisbewegung kann mittels einer entsprechenden mechanischen Vorrichtung, beispielsweise mittels einer Drehscheibe erfolgen.

Wie bereits erwähnt, ist es besonders wichtig, daß bei der Verifikation und Korrektur der Modulation des energiereichen Strahls die Form und Lage von Risikoorganen berücksichtigt wird. In entsprechender Weise ist also die Steuerung, das Verfahren und das Computerprogramm zur Vornahme der Steuerung auszubilden. Gerade in dieser Hinsicht weist die Erfindung besonders hohe Vorzüge gegenüber den bekannten Vorrichtungen und Verfahren auf, da die räumliche Erfassung und Verifikation die Gefahr großer Schäden durch Lage- und Anatomieveränderungen erheblich reduziert. Um die vorgenannte Verifikation innerhalb kürzester Zeit unter Berücksichtigung einer dreidimensionalen Momentaufnahme vornehmen zu können, wird vorgeschlagen, daß von dem Röntgenstrahl in den Sendepausen des energiereichen Strahls ein Teilbereich des Zielvolumens mit einem angrenzenden Bereich eines Risikoorgans erfaßt und zeitnah für die Verifikation berücksichtigt wird. Dadurch werden die zu verarbeitenden Daten auf den kritischen Bereich beschränkt, dadurch erheblich reduziert und trotzdem wird dort, wo es darauf ankommt, eine ständige, exakte Überprüfung im dreidimensionalen Raum vorgenommen.

Zweckmäßig ist es, wenn ein Protokoll über die applizierte Strahlung, vorzugsweise im dreidimensionalen Raum, erstellt wird und/oder ein Protokoll über die Korrekturen des Behandlungsplans für die durchgeführte Applikation der Strahlung.

Die vorgenannten Merkmale sind als Vorrichtung, als Computerprogramm oder als Steuerungsverfahren realisierbar. Das Computerprogramm ist selbstverständlich nur eine, wenn auch bevorzugte Ausführungsform, einer in der Weise niedergelegten Maschinensteuerungsabfolge, daß sie durch die Steuerung maschinell ausführbar ist. Diese könnte auch als Hardware oder in anderer Form maschinell ausführbar niedergelegt sein.

Bezüglich der Vorrichtung wird als vorteilhafte Weiterbildung vorgeschlagen, daß beide Medien, das zur Erfassung des energiereichen Strahls und das zur Erfassung des Röntgenstrahls, als ein Medium ausgebildet werden. Auf diese Weise wird ein Erfassungsmedium eingespart, was den gerätetechnischen Aufwand reduziert. Auch die Zuordnung der beiden Erfassungen wird vereinfacht. Dabei lassen sich die einzelnen Erfassungselemente auch für die Erfassung beider Strahlen einsetzen, indem die Röntgenstrahlen beispielsweise unmittelbar an der Oberfläche und der Therapiestrahl bei der Durchstrahlung des Mediums erfaßt wird. Das Medium muß aus einem Material bestehen, das durch den harten Therapiestrahl nicht beschädigt wird. Beispielsweise kann das Medium ein Array von Fotodioden sein, wobei letztere aus einem amorphem Material bestehen, beispielsweise aus amorphem Silizium oder amorphem Selen. Bei diesen kann es zu keiner Zerstörung einer Gitterstruktur kommen. Weiterhin sollten die Fotodioden in einem Gehäuse angeordnet sein, das nur eine geringe Schwächung des energiereichen Strahls zur Folge hat und zwar derart, daß die Applikation keine für die Behandlung relevanten Differenzen aufweist. Beispielsweise können die Fotodioden in einem Kunststoffgehäuse angeordnet werden, so daß es zu keiner nennenswerten Schwächung oder Streuung der Strahlen kommt.

Der Verdeutlichung der Erfindung dienen die nachfolgenden Erläuterungen anhand der Zeichnung. Es zeigen
- **Fig. 1**: eine Prinzipskizze eines Ausführungsbeispiels der erfindungsgemäßen Vorrichtung,
- **Fig. 2**: eine erfindungsgemäße Vorrichtung im Einsatz und
- **Fig. 3**: eine Erläuterung des Prinzips einer optimalen Bestrahlung, welche verifiziert werden soll.

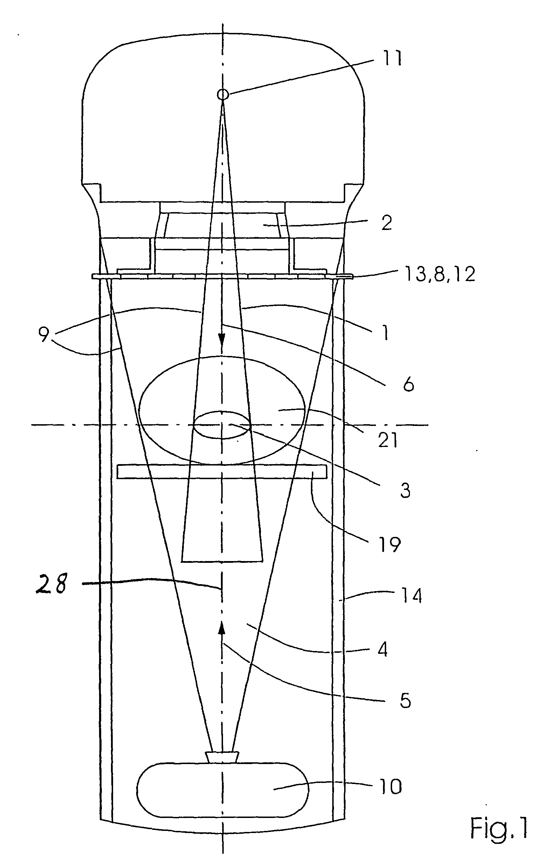

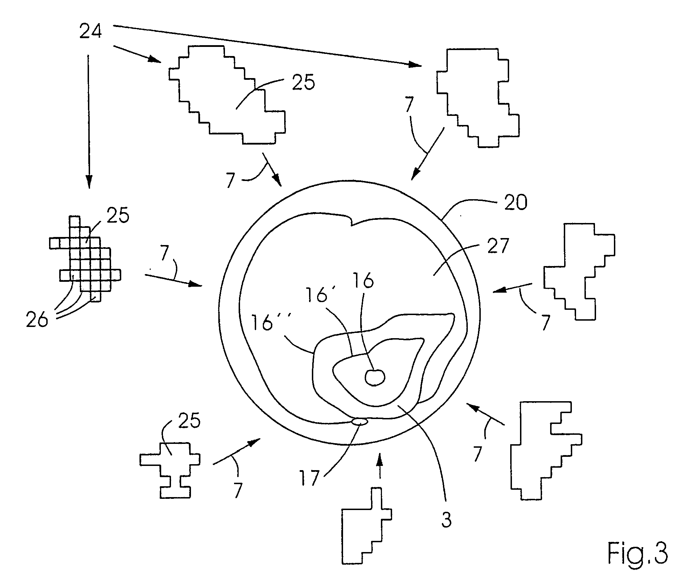

**Fig. 1** zeigt das Prinzip der Erfindung an einem Ausführungsbeispiel der erfindungsgemäßen Vorrichtung. Ein energiereicher Strahl 1 wird von einer Strahlenquelle 11 erzeugt und von einer Einrichtung zur Strahlenmodulation 2, beispielsweise einem Multileafkollimator, entsprechend dem Behandlungsplan moduliert und auf ein Zielvolumen 3 gerichtet. In der Regel ist dies ein Tumor eines Patienten 21, der auf einem Patiententisch 19 liegend behandelt wird. Zwischen der Einrichtung 2 zur Strahlenmodulation und dem Patienten 21 wird erfindungsgemäß im Strahlengang 9 ein Medium 8 zur Erfassung des energiereichen modulierten Strahls 1 bezüglich seiner Gestalt und bezüglich von Bereichen 16, 16', 16" unterschiedlicher Bestrahlungsdosis (siehe Fig. 3) angeordnet, damit die Formgebung und Intensitätsmodifikationen durch die Einrichtung 2 zur Strahlenmodulation erfaßt und überwacht werden können. Weicht die Modulation des Strahls 1 von ihrem Sollwert ab, kann eine Abschaltung oder eine Korrektur erfolgen.

Gegenüber der Strahlenquelle 11 für dem energiereichen Strahl 1 ist eine Strahlenquelle 10 für einen Röntgenstrahl 4 angeordnet. Die Anordnung erfolgt derart, daß ein Strahlengang 9 entsteht, bei dem die Richtung 5 des Röntgenstrahls 4 der Richtung 6 des energiereichen Strahls 1 im wesentlichen entgegengesetzt ist. Der Röntgenstrahl 4 dient der Erfassung des Zielvolumens 3 und der Anatomie und Lage des Patienten 21 in der bereits oben beschriebenen Art und Weise. Zur Erfassung des Röntgenstrahls 4 ist nach dem Hindurchtreten desselben durch den Patienten 21 ein Medium 12 angeordnet. Zweckmäßigerweise sind jedoch die Medien 8 und 12 als ein Medium 13 zur Erfassung des energiereichen Strahls 1 und des Röntgenstrahls 4 ausgebildet. Bezüglich einer zweckmäßigen Ausgestaltung wird auf die obigen Ausführungen verwiesen.

Die Anordnung der Strahlenquellen 11 und 10 erfolgt derart, daß durch den Therapiestrahl 1 das Zielvolumen 3 erfaßt wird und durch den Röntgenstrahl 4 das Zielvolumen 3 und dessen Umgebung, die bei der Modulation des Therapiestrahls 1 ebenfalls berücksichtigt werden sollte. Aus diesem Grund ist der Röntgenstrahl 4 weiter auseinanderlaufend gezeichnet als der Therapiestrahl 1, wobei er natürlich auch schmäler als gezeichnet ausgebildet sein kann, also nicht den gesamten Patienten 21 erfassen muß.

Wird ein Erfassungsmedium 13 vorgesehen, so muß dessen Fläche derart bemessen sein, daß diese die konisch auseinanderlaufenden Strahlen 1 und 4 in der Position der Anordnung des Erfassungsmediums 13 erfaßt.

Die Durchführung einer Behandlung erfolgt zweckmäßigerweise in folgenden Schritten:

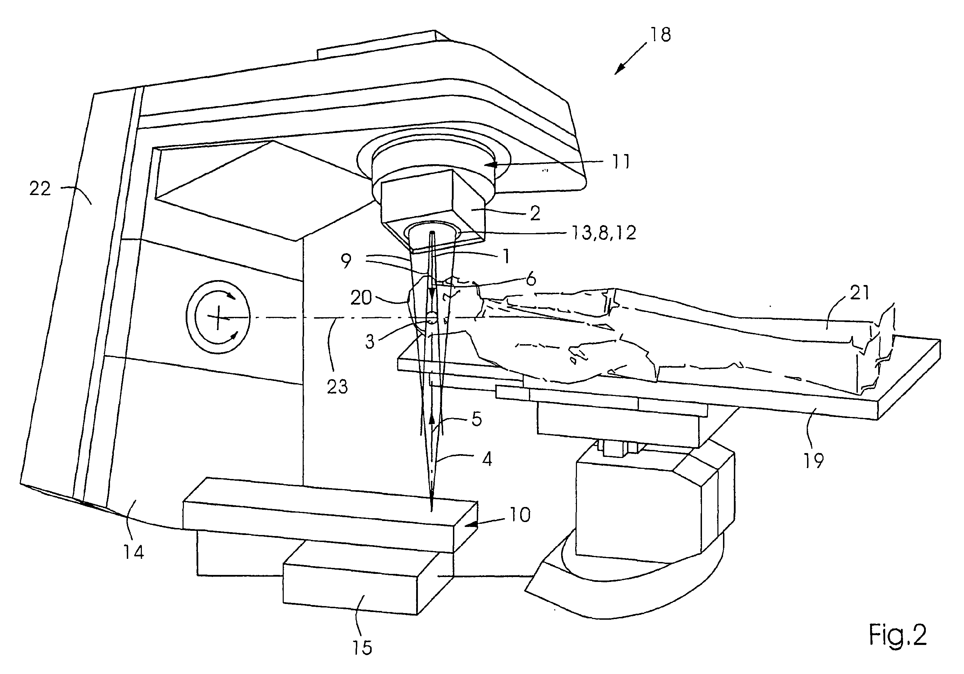

In einem ersten Schritt der Verifikationsprozedur wird unmittelbar vor Beginn der Strahlentherapie mit Hilfe des Computer-Tomographie-Systems, also des Röntgenstrahls 4 und eines Mediums 12 oder 13, ein aktueller Computer-Tomographie-Datensatz vom Patienten 21 in Therapiesituation gewonnen. Hiermit lassen sich Veränderungen in der Zielregion 3 und Lagerungsfehler des Patienten 21 direkt erkennen, so daß die nachfolgende Therapie auf diese neuen Daten abgestimmt werden kann. Diese Erfassung der Zielregion 3 sowie ihres Umfeldes erfolgt mehrmals aus verschiedenen Richtungen 7 (siehe Fig. 3), wobei diese Richtungen 7 durch eine Drehung der Gantry 14 in verschiedene Positionen erzielt werden. Durch die so gewonnenen Daten kann eine Steuerung (siehe Fig. 2) ein dreidimensionales Bild des Zielvolumens 3 und seines Umfeldes erstellen und dies mit einem eingegebenen, vorerstellten dreidimensionalen Behandlungsplan vergleichen. Danach können Korrekturen der Lage des Patienten 21, zum Beispiel durch Stellbewegungen des Patiententisches 19 (siehe Fig. 2), oder durch eine Korrektur des Behandlungsplans erfolgen.

In einem zweiten Schritt wird während der Applikation der Therapiestrahlenfelder 24 (siehe Fig. 3) die Feldform und die Intensitätsverteilung des Therapiestrahls 1 gemessen und protokolliert. Damit und auf der Basis des aktuellen Computer-Tomographie-Datensatzes kann die dem Patienten 21 applizierte Strahlendosisverteilung 16, 16', 16" (siehe Fig. 3) rekonstruiert und online verifiziert werden. Gegebenenfalls kann die Strahlenapplikation bei etwaigen Abweichungen unterbrochen bzw. mit entsprechenden unmittelbaren Korrekturen fortgesetzt werden. Durch die Art der Anordnung der Röntgenstrahlquelle 10 und des Mediums 13 zur Erfassung der Strahlen 1 und 4 ist es auch möglich, die relative Lage von Strukturen (Zielvolumen 3, mit unterschiedlicher Dosis zu bestrahlende Bereiche 16, 16', 16" des Zielvolumens 3 und Risikoorgane 17) mit niedrigem Kontrast (Weichteilkontrast) im Therapiestrahlenfeld 24 und dessen Umgebung (siehe Fig. 3) mit Hilfe des Röntgenstrahls 4 während der Applikation der einzelnen Therapiestrahlenfelder 24 zu überwachen und wenn möglich, eine unverzügliche, möglichst fast zeitgleiche Korrektur vorzunehmen.

Dabei ist eine laufende Erfassung der genannten Gegebenheiten notwendig, die erfindungsgemäß in den Sendepausen des energiereichen Strahls 1 mittels des Röntgenstrahls 4 und dem Medium 12 oder 13 erfolgt und unmittelbar für die folgende Applikation berücksichtigt werden kann. Auch diese Erfassung wird in die vorerfaßten dreidimensionalen Gegebenheiten einbezogen, um eine exakte Verifikation und Korrektur zu erzielen.

Besonders vorteilhaft ist es, wenn auch die vorgenannten laufend erfolgenden "Momentaufnahmen" des Zielvolumens 3 während der Sendepausen des energiereichen Strahls 1 dreidimensionale Gegebenheiten erfassen. Dies ist möglich, indem der Röntgenstrahl 4 während dieser Erfassung aus verschiedenen Richtungen auf das Zielvolumen 3 gerichtet wird. Als ein Ausführungsbeispiel wird vorgeschlagen, daß die Strahlenquelle 10 für den Röntgenstrahl 11 derart ausgebildet ist, daß sie eine Kreisbewegung in einer Ebene um eine durch das Zielvolumen 3 zur Strahlenquelle 11 des energiereichen Strahls 1 führenden Achse 28 vollziehen kann. Diese ist nicht eingezeichnet, da sie sehr klein ist, so daß die Richtungen 5, 6 der Strahlen 1, 4 noch im wesentlichen entgegengesetzt gerichtet sind. Selbstverständlich muß dann auch die Steuerung 15 (siehe Fig. 2) derart eingerichtet sein, daß sie mittels der Daten der Röntgenaufnahmen auf dieser Kreisbewegung eine dreidimensionale Darstellung des Bereichs des Zielvolumens 3 erstellt und diese zur Verifikation heranzieht.

Die Einrichtung zur Erzeugung dieser meist in einem kleinen Bereich vorzunehmenden Kreisbewegung kann beispielsweise mechanisch durch außermittige Anordnung der Strahlenquelle 10 des Röntgenstrahls 4 auf einer Drehscheibe erfolgen.

Die vorgenannten "Momentaufnahmen" können sich auf einen kritischen Bereich beschränken, beispielsweise auf einen Bereich, in dem der Tumor an ein Risikoorgan grenzt.

**Fig. 2** zeigt eine erfindungsgemäße Vorrichtung im Einsatz. Es handelt sich dabei um einen üblichen Aufbau eines Bestrahlungsgeräts 18 mit einer Strahlenquelle 11 für den Therapiestrahl 1, einem Patiententisch 19 und einer Einrichtung 2 zur Strahlenmodulation, um die medizinisch indizierte Strahlung auf ein Zielvolumen 3, beispielsweise am Kopf 20 eines Patienten 21, so zu richten, daß ein Tumor maximal geschädigt und das umliegende Gewebe maximal geschont wird. Zu diesem Zweck ist ein Gestell (Gantry) 14 vorgesehen, das den Patienten 21 allseitig umkreisen kann. Die Gantry 14 enthält die Strahlenquelle 11 für den Therapiestrahl 1, wobei die energiereiche Strahlung 1, beispielsweise durch einen Linearbeschleuniger 22, erzeugt wird. Der Strahlenquelle 11 gegenüberliegend ist auf der Gantry 14 die Strahlenquelle 10 für den Röntgenstrahl 4 in der bereits zu Fig. 1 beschriebenen Weise angeordnet. Diesbezüglich wird auf die obige Beschreibung verwiesen, wobei gleiche Bezugszeichen funktionsidentische Bauteile bezeichnen.

Die Gantry 14 ist um eine horizontale Rotationsachse 23 drehbar, wobei die Strahlen 1 und 4 auf das Zielvolumen 3 beziehungsweise dessen Umgebung gerichtet sind. Das Zielvolumen 3 befindet sich im Isozentrum der Strahlen 1 und 4, wobei die Strahlenquellen 11 und 10 und eine Einrichtung 2 zur Strahlenmodulation durch die Rotation der Gantry 14 um die Achse 23 den Patienten 21 umkreisen. Gleichzeitig kann eine Verschiebung oder Drehung des Behandlungstisches 19 stattfinden, um eine exakte Einstellung der Einstrahlung des Therapiestrahls 1 auf das Zielvolumen 3 des Patienten 21 vorzunehmen. Es kann damit auch die Lage des Patienten 21 so korrigiert werden, daß er gemäß dem Behandlungsplan positioniert ist.

Der Zweck einer solchen Gantry besteht darin, daß durch die unterschiedlichen Bestrahlungsrichtungen 7 (siehe Fig. 3) das Zielvolumen 3 eine maximale Bestrahlung erfährt, jedoch das umliegende Gewebe maximal geschont wird, da es immer nur kurzzeitig den energiereichen Strahlen 1 ausgesetzt ist. Außerdem ist es oft erforderlich, daß bestimmte Bereiche des Körpers, wie beispielsweise das Rückenmark oder andere Risikoorgane 17 von der energiereichen Strahlung 1 möglichst völlig verschont werden, also durch die Ausgestaltung der Therapiestrahlenfelder 24 aus den verschiedenen Richtungen 7 (siehe Fig. 3) möglichst weitgehend ausgespart sind.

Die Lage und das Profil des Zielvolumens 3 sowie die Lage von Risikoorganen 17 oder von Bereichen 16, 16', 16", die für unterschiedliche Bestrahlungsdosen vorgesehen sind, wird vom Medium 13 mit Hilfe des Röntgenstrahls 4 - wie bereits beschrieben dreidimensional - erfaßt. Gleichzeitig wird auch der Ist-Zustand des modellierten Therapiestrahls 1 erfaßt und gegebenenfalls eine der beschriebenen Korrekturen vorgenommen. Diese Daten werden derart umgesetzt, daß der Kollimator 2 eine entsprechende Kollimatoröffnung ausbildet, wobei durch die erfindungsgemäße Erfassung und Verifikation die exakte Form des Zielvolumens 3 mit der gewünschten Strahlendosisverteilung 16, 16', 16" (siehe Fig. 3) bestrahlt werden kann. Bei einem Kollimator 2 wird die Strahlendosisverteilung 16, 16', 16" dadurch erzielt, daß auch aus mehreren Richtungen 7 ein oder mehrere Therapiestrahlenfelder 24 unterschiedlicher Zeitdauer appliziert werden.

Um alle Einstellungen vornehmen zu können, ist eine Steuerung 15, dies kann ein speziell ausgebildeter oder universell einsetzbarer Computer sein, vorgesehen. Die Steuerung 15 ist zur Aufnahme der zu verarbeitenden Daten und zur Steuerung der eingangs genannten Vorgehensweise mit den Medien 8 und 12 oder dem Medium 13 zur Erfassung der Strahlen 1 und 4, mit der Einrichtung 2 zur Modulation des energiereichen Strahls 1 mit einem Antrieb für die Einstellung der Lage des Patiententisches 19 sowie mit den Strahlenquellen 10 und 11, einem Antrieb und einer Stellungserfassung für die Gantry 14 verbunden und mit dem Behandlungsplan ausgestattet. Sie wird entsprechend dem offenbarten Steuerungsverfahren, beispielsweise mit Hilfe des erfindungsgemäßen Computerprogramms betrieben. Auf der Grundlage des Bestrahlungsplans und der oben beschriebenen laufenden Verifikation wird die Strahlenquelle 11 und die Einrichtung 2 zur Strahlenmodulation, die Gantry 14 und gegebenenfalls auch der Patiententisch 21 gesteuert. Bei der Einrichtung 2 kann es sich sowohl um einen Kollimator als auch um einen Scanner handeln. Die jeweils zu behandelnden Therapiestrahlenfelder 24 werden durch den Kollimator begrenzt oder durch Scannen eines Therapiestrahls 1 erzeugt.

**Fig. 3** zeigt eine Erläuterung des Prinzips einer Tumorbestrahlung, wobei die Applikation einer medizinisch indizierten energiereichen Strahlung 1 aus verschiedenen Richtungen 7 vorgenommen wird. Um ein zu bestrahlendes Zielvolumen 3, beispielsweise einen Tumor, in der bereits dargelegten Weise in optimaler Weise zu bestrahlen und das angrenzende Gewebe möglichst zu schonen, ist es erforderlich, daß verschiedene Therapiestrahlenfelder 24 für jede der verschiedenen Bestrahlungsrichtungen 7 ausgebildet werden. Dazu dient die Einrichtung 2 zur Strahlenmodulation, die als Kollimator oder als Scanner ausgebildet sein kann. Um zu erreichen, daß das zu bestrahlende Zielvolumen 3 die notwendige Dosis erhält, aber Risikoorgane 17 geschont werden, kann beispielsweise vorgesehen sein, daß die Therapiestrahlenfelder 24 als Matrizen 25 von Einzelfeldern 26 mit verschiedener Bestrahlungsdosis gebildet werden. Andere Möglichkeiten, wie ein kontinuierliches Abscannen, sind selbstverständlich denkbar. Derartige Matrizen 25 lassen sich durch die Leafverstellungen eines Multileafkollimators in fast jeder erdenklichen Form nachbilden, wobei durch dünne Leafs eine möglichst feine Nachbildung der zu bestrahlenden Therapiestrahlenfelder 24 erzielt wird. Zusätzlich zur Darstellung können aus einer Richtung 7 mehrere verschiedene Therapiestrahlenfelder 24 mit unterschiedlicher Zeitdauer appliziert werden, um in optimaler Weise Bereiche 16, 16', 16" mit einer unterschiedlichen Bestrahlungsdosis zu erzielen. Bei diesem Vorgang findet die erfindungsgemäße, nahezu zeitidentische Verifikation und Korrektur in bereits oben beschriebener Weise statt, also mit einer laufenden Verifikation, die bei jeder Bestrahlung aus einer Richtung 7 oftmals vorgenommen wird.

Die Darstellung der Figuren ist nur eine beispielhafte Darstellung der Erfindung. Denkbar wäre es auch, daß die Therapiestrahlenfelder 24 statt durch einen Kollimator durch einen Scanner erzeugt werden. Dann dient dieser als Einrichtung 2 zur Strahlenmodulation und das Medium 8 oder 13 muß die gescannten Therapiestrahlenfelder 24 erfassen, damit auf entsprechende Weise die erfindungsgemäße Verifikation sowie eine Korrektur, gegebenenfalls auch eine Unterbrechung der Behandlung erfolgen kann.

### Bezugszeichenliste

- 1: energiereicher modulierter Strahl (Therapiestrahl)
- 2: Einrichtung zur Strahlenmodulation
- 3: Zielvolumen
- 4: Röntgenstrahl
- 5: Richtung des Röntgenstrahls
- 6: Richtung des energiereichen Strahls
- 7: verschiedene Richtungen der Erfassung und Bestrahlung des Zielvolumens
- 8: Medium zur Erfassung des energiereichen modulierten Strahls
- 9: Strahlengang
- 10: Strahlenquelle für Röntgenstrahl
- 11: Strahlenquelle für energiereichen Strahl
- 12: Medium zur Erfassung des Röntgenstrahls
- 13: Medium zur Erfassung des energiereichen Strahls und des Röntgenstrahls
- 14: Gantry
- 15: Steuerung (Computer)
- 16, 16', 16": Bereiche unterschiedlicher Bestrahlungsdosis (Strahlendosisverteilung)
- 17: Risikoorgane (z. B. Rückenmark)
- 18: Bestrahlungsgerät
- 19: Patiententisch
- 20: Kopf
- 21: Patient
- 22: Linearbeschleuniger
- 23: Rotationsachse der Gantry
- 24: Therapiestrahlenfelder
- 25: Matrizen
- 26: Einzelfelder
- 27: Gehirn
- 28: Achse durch das Zielvolumen zur Strahlenquelle des energiereichen Strahls verlaufend

## Patentansprüche

1. Vorrichtung zur Durchführung und Verifikation einer therapeutischen Bestrahlung, die eine Strahlenquelle (11) für einen energiereichen Strahl (1) und eine Einrichtung (2) zur Modulation des energiereichen Strahls (1) auf der Gantry (14) eines Bestrahlungsgeräts aufweist, wobei für die Verifikation eine Strahlenquelle (10) für einen Röntgenstrahl (4) im Verhältnis zu einem Zielvolumen (3) der Strahlenquelle (11) für den energiereichen Strahl (1) derart gegenüberliegend angeordnet ist, daß die Richtungen (5, 6) der Strahlen (1, 4) im wesentlichen entgegengesetzt sind und wobei ein Medium (12, 13) zur Erfassung des Röntgenstrahls (4) bezüglich der Richtung (5) desselben hinter dem Zielvolumen (3) und ein Medium (8, 13) zur Erfassung des energiereichen Strahls (1), bezüglich der Richtung (6) dieses Strahls (1) vor dem Zielvolumen (3) angeordnet sind,
wobei das Medium (8, 13) zur Erfassung von Bereichen (16, 16', 16") unterschiedlicher Bestrahlungsdosis des energiereichen Strahls (1) ausgebildet ist
**dadurch gekennzeichnet, daß** eine Steuerung (15) mit den Medien (8, 12 oder 13) zur Erfassung der Strahlen (1 und 4), mit der Einrichtung (2) zur Modulation des energiereichen Strahls (1), mit einem Antrieb für die Einstellung der Lage des Patiententisches (19) und mit den Strahlenquellen (10 und 11) verbunden, mit einem Behandlungsplan ladbar und derart eingerichtet ist, daß sie die Gantry (14) und die vorgenannten Elemente (8, 12, 2, 10 und 11) in der Weise steuert, daß
a) vor der Applikation des energiereichen Strahls (1) eine räumliche Erfassung der Anatomie und Lage des Patienten (21) im Bereich des Zielvolumens (3) durch den Röntgenstrahl (4) erfolgt, indem dieser aus verschiedenen Richtungen (7) auf diesen Bereich gerichtet wird,
b) die erfaßte Anatomie und Lage des Patienten (21) mit dem Behandlungsplan verglichen und die Patientenposition und/oder der Behandlungsplan gegebenenfalls korrigiert wird,
c) der energiereiche Strahl (1) aus einer ersten Richtung (7) appliziert und dabei bezüglich seiner Form und den Bereichen (16, 16', 16") unterschiedlicher Bestrahlungsdosis erfaßt wird,
d) in einer Sendepause des energiereichen Strahls (1) eine Erfassung von mindestens einem Teilbereich des Zielvolumens (3) mit der allernächsten Umgebung durch den Röntgenstrahl (4) erfolgt,
e) die Röntgenaufnahme mit dem erfaßten applizierten energiereichen Strahl (1) verglichen und der Behandlungsplan gegebenenfalls korrigiert wird,
f) die Schritte c), d) und e) bis zur Erreichung der durch den Behandlungsplan für die erste Bestrahlungsrichtung (7) vorgeschriebenen Applikation wiederholt werden,
g) die Schritte c) bis f) für alle im Behandlungsplan vorgesehenen Bestrahlungsrichtungen (7) wiederholt werden.

2. Vorrichtung nach Anspruch 1,
**dadurch gekennzeichnet,**
**daß** sie derart ausgebildet ist, daß der Röntgenstrahl (4) in den Sendepausen des energiereichen Strahls (1) aus verschiedenen Richtungen, jedoch innerhalb eines so kleinen Bereichs, daß er der Richtung (6) der Strahlen (1) des energiereichen Strahls (1) noch im wesentlichen entgegengesetzt ist, auf mindestens einen Teilbereich des Zielvolumens (3) mit allernächster Umgebung desselben gerichtet wird, um durch diese aus verschiedenen Richtungen erfaßten Daten auch den vorgenannten Erfassungsbereich dreidimensional zu erfassen und zeitnah für die Verifikation zu berücksichtigen.

3. Vorrichtung nach Anspruch 2,
**dadurch gekennzeichnet,**
**daß** die Strahlungsquelle (10) für den Röntgenstrahl (11) derart ausgebildet ist, daß sie eine Kreisbewegung in einer Ebene, die um eine durch das Zielvolumen (3) zur Strahlungsquelle (11) des energiereichen Strahls (1) führenden Achse (28) angeordnet ist, vollzieht.

4. Vorrichtung nach Anspruch 1, 2 oder 3,
**dadurch gekennzeichnet,**
**daß** die Steuerung (15) derart ausgebildet ist, daß sie bei der Verifikation und Korrektur der Modulation des energiereichen Strahls (1) die Form und Lage von Risikoorganen (17) berücksichtigt.

5. Vorrichtung nach einem der Ansprüche 1 bis 4,
**dadurch gekennzeichnet,**
**daß** von dem Röntgenstrahl (4) in den Sendepausen des energiereichen Strahls (1) ein Teilbereich des Zielvolumens (3) mit einem angrenzenden Bereich eines Risikoorgans (17) erfaßbar und zeitnah für die Verifikation berücksichtigbar ist.

6. Vorrichtung nach einem der Ansprüche 1 bis 5,
**dadurch gekennzeichnet,**
**daß** die Steuerung zur Erstellung eines Protokolls über die applizierte Strahlung (1) ausgebildet ist.

7. Vorrichtung nach Anspruch 6,
**dadurch gekennzeichnet,**
**daß** die Steuerung (15) für die Erstellung eines Protokolls im dreidimensionalen Raum ausgebildet ist.

8. Vorrichtung nach einem der Ansprüche 1 bis 7,
**dadurch gekennzeichnet,**
**daß** die Steuerung (15) zur Erstellung eines Protokolls über die Korrekturen des Behandlungsplans für die durchgeführte Applikation der Strahlung (1) ausgebildet ist.

9. Vorrichtung nach einem der Ansprüche 1 bis 8,
**dadurch gekennzeichnet,**
**daß** die Medien (8, 12) als ein Medium (13) zur Erfassung des energiereichen Strahls (1) und des Röntgenstrahls (4) ausgebildet sind.

10. Vorrichtung nach Anspruch 9,
**dadurch gekennzeichnet,**
**daß** das Medium (13) ein Array von aus amorphem Material bestehenden Fotodioden ist.

11. Vorrichtung nach Anspruch 10,
**dadurch gekennzeichnet,**
**daß** die Fotodioden in einem Gehäuse angeordnet sind, das nur eine geringe Schwächung des energiereichen Strahls (1) zur Folge hat.

12. Computerprogramm zur Steuerung einer Vorrichtung nach einem der Ansprüche 1 bis 6, 8.

## Claims

1. A device for the execution and verification of therapeutic irradiation, comprising a source of radiation (11) for a high-energy beam (1) and means (2) for modulating said high-energy beam (1) disposed on the gantry (14) of an irradiating device, wherein, for the purpose of verification, a source of radiation (10) for an X-ray beam (4) is disposed opposite to said source of radiation (11) for the high-energy beam (1) in relation to a target volume (3) in such a manner that the beams (1, 4) are emitted in substantially opposite directions (5, 6) and wherein a medium (12, 13) for the acquisition of the X-ray beam (4) is disposed behind of the target volume (3) as regarded in the direction (5) of said X-ray beam and a medium (8, 13) for the acquisition of the high-energy beam (1) is disposed in front of the target volume (3), as regarded in the direction (6) of said beam (1), wherein said medium (8, 13) is adapted for the acquisition of regions (16, 16', 16") requiring different irradiation doses of the high-energy beam (1),
**characterized in that**
a control system (15) comprising media (8, 12, or 13) for the acquisition of the beams (1 and 4), is linked with said means (2) for modulating said high-energy beam (1), with a drive for setting the position of the patient's table (19), and with said sources of radiation (10 and 11), is capable of being loaded with a treatment schedule, and is designed in such a manner that it controls the gantry (14) and the aforementioned elements (8, 12, 2, 10, and 11) such that
a) prior to application of the high-energy beam (1), spatial acquisition of the anatomy and position of the patient (21) in the region of the target volume (3) is effected by the X-ray beam (4) by directing it toward this region from different directions (7),
b) the acquired anatomy and position of the patient (21) is compared with the treatment schedule, and the position of the patient and/or the treatment schedule are corrected, if appropriate,
c) the high-energy beam (1) is applied from a first direction (7) and at the same time recorded with regard to its shape and the regions (16, 16', 16") requiring different irradiation doses,
d) in an off-period of the high-energy beam (1) the acquisition of at least one subregion of the target volume (3) and its immediate surroundings is carried out by the X-ray beam (4),
e) the X-ray radiograph is compared with the recorded applied high-energy beam (1) and the treatment schedule is corrected, if appropriate,
f) the steps c), d) and e) are repeated until the application specified by the treatment schedule for the first direction of irradiation (7) has been achieved,
g) the steps c) to f) are repeated for all directions of irradiation (7) defined in the treatment schedule.

2. The device according to claim 1,
**characterized in that**
it is designed such that the X-ray beam (4), in the off-periods of the high-energy beam (1) coming from different directions, but within such a small region that it is still substantially opposite to the direction (6) of the rays (1) of the high-energy beam (1), is directed toward at least one subregion of the target volume (3) and its immediate surroundings in order to detect also the aforementioned acquisition region three-dimensionally by way of said data collected from different directions and to take it into consideration for verification in near real time.

3. The device according to claim 2,
**characterized in that**
said radiation source (10) for said X-ray beam (11) is formed such that it completes a circular movement in a plane disposed about an axis (28) passing through the target volume (3) toward the radiation source (11) for said high-energy beam (1).

4. The device according to claims 1, 2, or 3,
**characterized in that**
said control system (15) is adapted to allow for the shape and position of high-risk organs (17) during verification and correction of the modulation of said high-energy beam (1).

5. The device according to any one of claims 1 to 4,
**characterized in that**
said X-ray beam (4) can, in the off-periods of the high-energy beam (1), detect a subregion of the target volume (3) and an adjoining region of a high-risk organ (17) and can allow therefor for verification in near real time.

6. The device according to any one of claims 1 to 5.
**characterized in that**
said control system is adapted to make a protocol concerning the applied radiation (1).

7. The device according to claim 6,
**characterized in that**
said control system (15) is adapted to make a protocol in three-dimensional space.

8. The device according to any one of claims 1 to 7,
**characterized in that**
said control system (15) is adapted to make a protocol concerning the corrections of the treatment schedule effected for the applied radiation (1).

9. The device according to any one of claims 1 to 8,
**characterized in that**
said media (8, 12) are in the form of a medium (13) for the acquisition of the high-energy beam (1) and of the X-ray beam (4).

10. The device according to claim 9,
**characterized in that**
said medium (13) is an array of photodiodes consisting of amorphous material.

11. The device according to claim 10,
**characterized in that**
said photodiodes are disposed in a casing which causes only low attenuation of the high-energy beam (1).

12. A computer application for controlling a device according to any one of claims 1 to 6 and 8.

## Revendications

1. Dispositif pour la mise en oeuvre et la vérification d'une radiation thérapeutique qui présente une source de rayon (11) pour un rayon riche en énergie (1) et un dispositif (2) pour la modulation du rayon riche en énergie (1) sur la Gantry (14) d'un appareil de radiation, pour la vérification une source de rayon (10) pour un rayon X (4) étant disposée par rapport à un volume cible (3) de la source de rayon (11) pour le rayon riche en énergie (1)de manière opposée de sorte que les directions (5, 6) des rayons (1, 4) sont sensiblement opposées, et un support (12, 13) pour la saisie du rayon X (1) par rapport à la direction (5) de ce dernier étant placé derrière le volume cible (3) et un support (8, 13) pour la saisie du rayon riche en énergie (1) étant placé derrière le volume cible (3)par rapport à la direction (6) de ce rayon (1),
le support (8, 13) étant réalisé pour la détection de zones (16, 16', 16") de dose de rayonnement différente de u rayon riche en énergie (1), **caractérisé en ce qu'**une commande (15) est reliée aux supports (8, 12 ou 13) pour la détection des rayons (1 et 4), avec le dispositif (2) pour la modulation du rayon riche en énergie (1), avec un entraînement pour le réglage de la position de la table du patient (19) et avec la source de rayon (10 et 11), peut être chargée avec un plan de thérapie et être agencée de sorte qu'elle commande la Gantry (14) et les éléments précités (8, 12, 2, 10 et 11) de sorte qu'
a) avant l'application du rayon riche en énergie (1), une détection spatiale de l'anatomie et de la position du patient (21) s'effectue dans la zone du volume cible (3) par le rayon X (4) dans le fait que celui est dirigé sur cette zone depuis différentes directions (7),
b) l'anatomie et la position détectées du patient (21) sont comparées avec le plan de thérapie et la position de patient et/ou le plan de thérapie est éventuellement corrigé,
c) le rayon riche en énergie (1) est appliqué depuis une première direction (7) et est détecté en fonction de sa forme et des zones (16, 16', 16") de doses de rayonnement différentes,
d) pendant une pause d'émission du rayon riche en énergie (1) une détection d'au moins une zone partielle du volume cible (3) avec l'environnement le plus proche s'effectue par le rayon X (4),
e) l'enregistrement de rayon X est comparé avec le rayon riche en énergie appliqué saisi (1) et le plan de thérapie est éventuellement corrigé,
f) les étapes c), d) et e) sont répétées jusqu'à obtention de l'application prescrite par le plan de thérapie pour la première direction de rayonnement (7),
g) les étapes c) à f) sont répétées pour toutes les directions de rayonnement (7) prévues dans le plan de thérapie.

2. Dispositif selon la revendication 1,
**Caractérisé en ce qu'**il est conçu de sorte que le rayon X (4) est dirigé dans les pauses d'émission du rayon riche en énergie (1) depuis différents directions cependant dans une région suffisamment petite pour qu'elle soit encore essentiellement opposée à la direction (6) des rayons (1) du rayon riche en énergie (1), sur au moins une zone partielle du volume cible (3) avec l'environnement le plus proche de ce dernier pour saisir par ces données détectées provenant des différentes directions également la zone de saisie précitée de manière tridimensionnelle et d'en tenir compte en temps réel pour la vérification.

3. Dispositif selon la revendication 2, **caractérisé en ce que** la source de rayon (10) pour le rayon X (11) est réalisée de sorte qu'elle effectue un déplacement circulaire dans un plan qui est disposé autour d'un axe (28) menant à travers le volume cible (3) en direction de la source de rayon (11) du rayon riche en énergie (1).

4. Dispositif selon la revendication 1, 2 ou 3, **caractérisé en ce que** la commande (15) est réalisée de sorte qu'elle tient compte lors de la vérification et de la correction de la modulation du rayon riche en énergie (1) de la forme et de la position d'organes à risque (17).

5. Dispositif selon l'une des revendications 1 à 4, **caractérisé en ce que** le rayon (4) peut saisir dans les pauses d'émission du rayon riche en énergie (1) une zone partielle du volume cible (3) avec une zone attenante d'un organe à risque (17) et peut être pris en compte pour la vérification en temps réel.

6. Dispositif selon l'une des revendications 1 à 5, **caractérisé en ce que** la commande est réalisée pour l'établissement d'un protocole sur le rayonnement appliqué (1).

7. Dispositif selon la revendication 6, **caractérisé en ce que** la commande (15) pour l'établissement d'un protocole est réalisée dans un espace tridimensionnel.

8. Dispositif selon l'une des revendications 1 à 7, **caractérisé en ce que** la commande (15) est réalisée pour l'établissement d'un protocole sur les corrections du plan de thérapie pour l'application effectuée du rayonnement (1).

9. Dispositif selon l'une des revendications 1 à 8, **caractérisé en ce que** les supports (8, 12) sont réalisés comme des supports (139 pour la détection du rayon riche en énergie (1) et du rayon X (4).

10. Dispositif selon la revendication 9, **caractérisé en ce que** le support (13) est un réseau de photodiodes se composant de matériau amorphe.

11. Dispositif selon la revendication 10, **caractérisé en ce que** les photodiodes sont disposées dans un coffret qui ne provoque qu'un affaiblissement réduit du rayon riche en énergie (1).

12. Programme informatique pour la commande d'un dispositif selon l'une des revendications 1 à 6, 8.
